**Europäisches Patentamt**

**European Patent Office**

**Office européen des brevets**

(11) Publication number: **0 121 277**
**A1**

(12) # EUROPEAN PATENT APPLICATION

(21) Application number: **84200412.9**

(22) Date of filing: **22.03.84**

(51) Int. Cl.³: **A 61 F 9/00**

(30) Priority: **25.03.83 IT 2031383**

(43) Date of publication of application:
**10.10.84 Bulletin 84/41**

(84) Designated Contracting States:
**AT BE CH DE FR GB LI LU NL SE**

(71) Applicant: **SIS-TER S.p.A.**
**Via Crema, 14**
**I-26020 Palazzo Pignano (Cremona)(IT)**

(72) Inventor: **Nardi, Giancarlo**
**Via O. Gentileschi 8**
**Pisa(IT)**

(74) Representative: **Dragotti, Gianfranco**
**c/o SAIC BREVETTI S.a.s. Via Spontini, 1**
**I-20131 Milano(IT)**

(54) **Automatic suction and pumping apparatus.**

(57) In the oculistic surgery a suction action is applied to the axial duct of a bistoury (16) by means of a suction unit (11) in which said axial duct is connected, through a suction line, to an adjustable vacuum source, and at the same time infusion liquid is introduced, under constant and adjustable pressure, through an infusion unit (12) comprising a liquid feed undergoing the constant and adjustable pressure of a thrust gas.

Fig.1

SPECIFICATION

The present invention relates to an automatic suction and pumping apparatus, particularly an apparatus for the drainage, infusion and pressurization of the eyeball. Thus in the following description, reference shall be made to such a specific use, without it being meant as a limitation of the use possibilities of the present invention. It is known that, in the field of the oculistic surgery, operations are carried out by means of bistouries or special needles, mounted on handles (handgrips), and possibly combined with optical fibre lightening devices.

These surgical instruments normally consist of a hollow point, having an internal axial duct, wherein a cutting member is seated and operated at an opening or window; through the axial duct and thus through said opening or window a suction action is applied whereby the cutting and reduction to fragments of intraocular materials takes place, the fragments being then removed through the same axial duct.

It is clear that at the same time of the suction action an infusion action must take place, namely the introduction for instance of physiological solution, in order to prevent the eyeball from being collapsed and to maintain at suitable values the endo-ocular pressure.

To date these simultaneous functions of suction and infusion under pressure have been fulfilled by using suction syringes connected to the said axial duct and devices for the introduction under adjustable pressure of physiological solution into the endo-ocular cavity.

These devices may be of the gravity type, with a feeding tank positionable at an adjustable height, the pressure adjustment being in this case, as it will be understood, definitely rough besides being not ready.

Alternatively said devices may comprise a pump, preferably a pump of peristaltic type. In this case, owing to the well known periodical irregularity of the suction and delivery phases, a discontinous and pulsing pressure is caused to occur within the

- 3 -

0121277

eyeball with not constant pressure levels.

These pumping devices, furthermore, may constitute a potential danger in the case of operative fault of the normal safety device and cause irremediable damages if the extreme delicacy of the subject organ, namely the eye, is considered.

It is to be noted that, still according to the prior art, the aforesaid functions of suction and of pressure infusion may be fulfilled by means of only one surgical instrument having suitable fittings, or by means of two separate instruments, namely one performing the cutting and reduction to fragments with suction action and the other performing the introduction of physiological solution under pressure.

Lastly a lightening device, preferably of the type comprising optical fibers, may be directly associated to the surgical instrument (if only one) or to the instrument and to the infusion needle, or even to a third independent needle.

The main purpose of the present invention is that of providing a complementary apparatus for a preferably surgical instrument, with respect to which simultaneous effects of suction and of introduction of a liquid under adjustable pressure must be achieved.

A more specific purpose of the present invention is that of providing an apparatus, complementary with respect to an instrument for intraocular surgery, capable of simultaneously fulfilling the functions of suction and drainage, as well as of infusion and maintenance of the endo-ocular pressure.

A particular purpose of the present invention is that of providing an apparatus of the aforesaid type capable of providing suction and pressurizing actions, with continous, stable and readily controllable pressure levels.

These purposes are achieved through an apparatus comprising a suction unit and an unit for the infusionunder pressure, characterized in that said suction unit comprises a connecting section to the outlet fitting of the surgical instrument adapted to give place to a continuous flow connection with the axial duct of said instrument, and a continuous and precisely adjustable suction source, whereas said unit for infusion under pressure comprises a feeding tank for the infusion liquid, means for feeding a thrust gas, under desired and adjustable pressure, to said feeding tank, whereby said liquid is discharged from said tank at the pressure of said thrust gas independently from the hydraulic head of said liquid with respect to the outlet from said feeding tank, connection means between said outlet and an element for the infusion under pressure, and means for detecting the pressure of said infusion liquid where it is discharged from said infusion element, said detecting means being connected to control and adjustment means of the pressure of said thrust gas fed to said feeding tânk.

According to a preferred embodiment of the apparatus according to the present invention said surgical instrument is a bistoury for intraocular surgery.

Moreover said connection section of said suction unit and said connecting element of said infusion unit are in form of disposable devices, whereby a perfectly sterile condition is ensured at the part mostly exposed to a contamination.

According to the preferred embodiment of the present invention said suction source consists of a hydraulic pump having the delivery side thereof connected to a collecting and feeding tank from which the suction duct of the same pump originates, the pump thus operating in a closed circuit over said tank, in the suction duct of said pump an adjustable neck being provided causing downstream thereof in said duct a reduced pressure, said connecting section to the axial duct of said surgical instrument being connected to the portion of said suction duct downstream of said neck, whereby in said section and in said axial duct a vacuum is caused to take place.

The peculiar features and advantages of the present invention shall more clearly appear from the following detailed description, referred to the accompanying drawings, in which:

fig. 1 is a schematic view of an eyeball with a surgical instrument in the operative position and an element for infusion under pressure, both connected to the apparatus according to the invention;

fig. 2 is a schematic view, on enlarged scale, of an embodiment of bistoury for intraocular surgery;

fig. 3 shows a scheme of the suction unit, and

fig. 4 shows a scheme of the unit for the infusion under pressure.

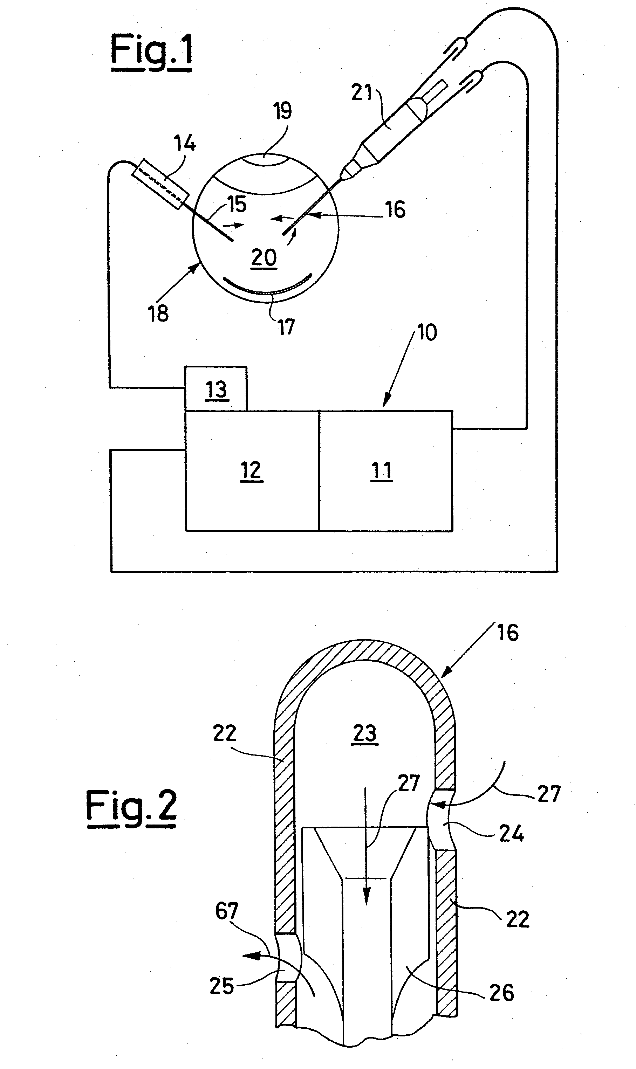

Referring firstly to the figs. 1 and 2, the apparatus according to the invention comprises a suction unit 11 and an unit 12 for the infusion under pressure.

In fig. 1 reference 13 does indicate the feed unit for a bundle of optical fibers 14, ending with a lightening needle 15.

Reference 16 generically indicates a bistoury for intra-ocular surgery, for instance for the cutting of corneal bindings which may involve the retina 17 of the eyeball 18, comprising the pupilla 19.

As clearly shown in fig. 1 the needle-like bistoury 16 is introduced in the endo-ocular cavity 20 and is connected to an handgrip 21.

As schematically shown on enlarged scale in fig. 2, the bistoury 16 comprises a needle-like outer casing 22, having an inner axial duct 23, in which a suction opening or window 24 and an opening 25 for the infusion under pressure are provided.

A cutting member 26 is housed in the inner axial duct 23, and is vertically reciprocable along the axis of the duct and actuated for instance from pulsating compressed air in a per se known manner.

It is understood that in this way the cutting edge of the member 26 passing before the opening 24 does cut bindings and other intra-ocular material as sucked into the said opening by the suction action applied to the duct 23.

At the same time, thanks to the said suction action, the fragments resulting from the cutting are conveyed along the same axial duct as indicated by the arrows 27.

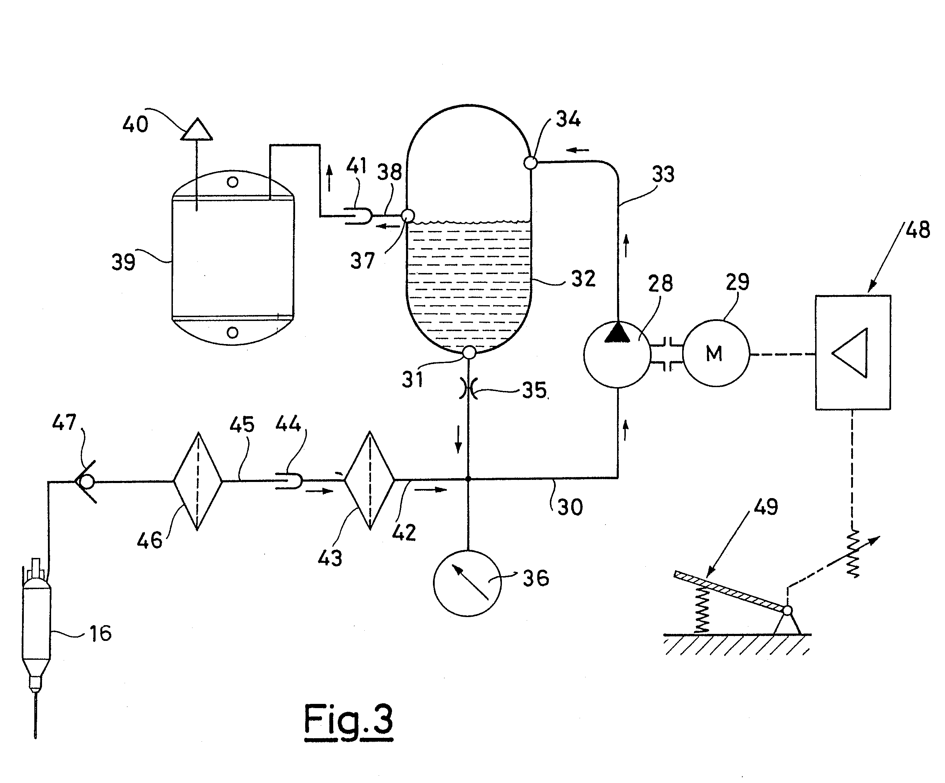

Turning now to the fig. 3, the suction unit is shown comprising a pump 28, driven by an electric motor 29. The suction side of the pump, as represented by the duct 30, is connected to the discharge outlet 31 of a tank 32, whereas the delivery duct 33 is connected to the same tank 32, at an upper inlet 34. The tank 32 contains water or other liquid, whereby the pump does return to the same tank the liquid sucked through the outlet 31 and the duct 30.

In the suction duct 30 an adjustable neck 35 is provided, it being schematically shown in the drawing, whereby downstream of the neck a reduced pressure is generated in the duct 30, it being measurable by means of the vacuometer 36.

In the tank 32 a further overflow discharge 37 is provided, at an intermediate location between the outlet 31 and the inlet 34, permitting the discharge of excess liquid entering the suction duct of the pump.

This excess is conveyed from the discharge 37 through the duct 38 to a collecting bag 39 having an air vent 40.

The duct 38 is preferably provided with a fitting 41 for the quick detachement of the bag 39.

Downstream of the neck 35 a duct 42 is connected to the duct 30, the duct 42 having a safety filter 43 and a fitting 44.

To the latter fitting the end of a suction line 45 is fastened, the other end being connected to the said axial duct 23 of the bistoury 16.

A filter 46 is provided in the line 45, whereas a check valve 47 prevents any flow towards the bistoury 16.

The suction line 45, with the related components (filter 46 and valve 47), is preferably of the sterile disposable type.

The operation of the above described suction unit is per se evident, since the vacuum generated in the duct 30 is acting also in the duct 42 and thus in the suction line 45.

Since the said vacuum is adjustable, also the suction action in the axial duct of the bistoury 16 can be controlled.

To this end, the motor 29 is provided with a control, per se known, for the adjustment of the rotation speed of the impeller of the pump 28, this control being indicated by the reference 48 and preferably of the electronic type, which in turn is controlled by a device 49 for the manual or foot operation.

It is lastly to be noted that in the course of the experimental tests it was observed that the vacuum which can be obtained in the duct 30 may attain very high values, even of the order of 700 Hg mm, with the self-evident operating advantages.

Of course the use of other systems useful for producing such a vacuum is foreseeable,

such as for instance an ejector device, connected to the duct 42.

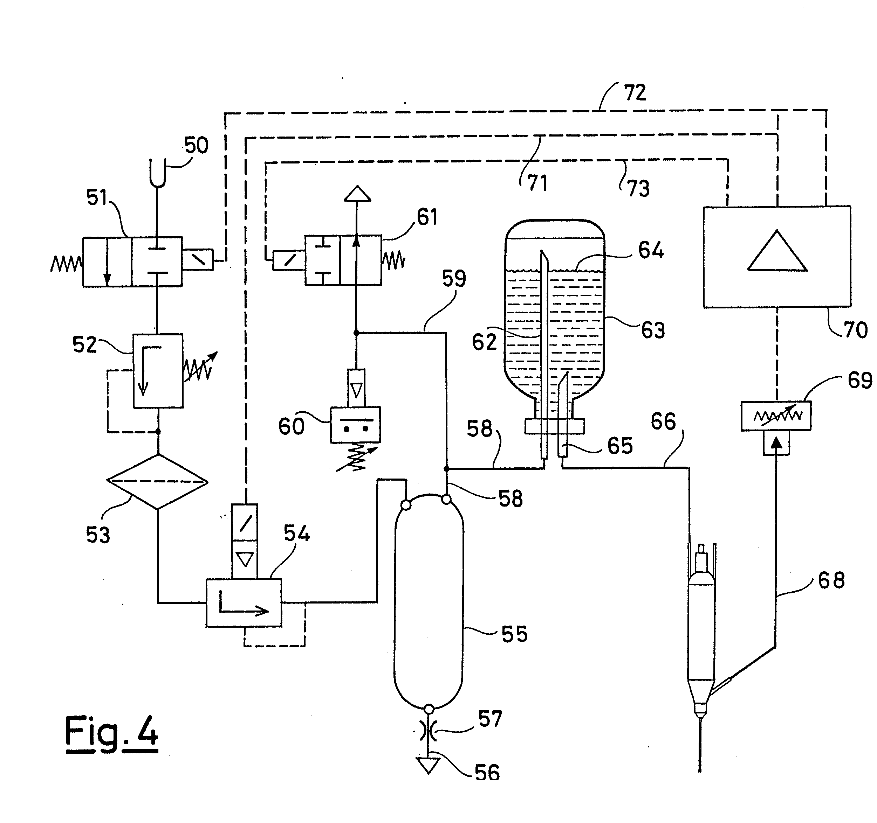

Considering now fig. 4, there is shown the unit for the infusion under pressure, comprising a fitting 50 for the inflow of air or other gas under pressure. The latter, through an electrovalve 51, arrives to the pressure control device 52, wherein the air pressure is firstly controlled and stabilized.

Then the air, passing through a filter 53, reaches an electropneumatic control device 54, by which the air pressure is very precisely adjusted.

From the device 54 the pressurized air is conveyed to a tank 55, having an outlet 56

with a neck 57. A connection line 58 originates from the tank 55, a safety line 59, comprising a pressure control switch 60 and a quick discharge electrovalve 61, being connected to the connecting line 58.

- 8 -

The connection line 58 does convey the air or other gas under pressure to a duct 62, housed within a plenum chamber 63, the latter serving for the feeding of infusion liquid, for instance physiological solution.

The discharge of the duct 62 is above the level 64 of the liquid in the plenum chamber 63, whereby in the space above the liquid a desired and constant pressure is established.

The plenum chamber 63 has a discharge 65 of the infusion liquid, said discharge being connected, through the line 66, to the handgrip and thus, in a per se known manner, to the infusion opening 25 (fig. 2), whereby the said liquid enters the endo-ocular cavity as indicated by the arrow 67, at the desired pressure and flowrate.

The bistoury 16 is furthermore connected to a pressure sensing device (not shown and of known type) by which, through the line 68 and a pressure transducer 69, a corresponding signal is sent to the electronic control unit having a comparator 70.

The latter, in a per se known manner, starting from the value measured by the aforesaid pressure sensing device, carries out the comparison with a predetermined theoretical value, giving a control signal (feed-back line 71) for the electropenumatic control device 54.

Such a control unit, moreover, depending on warning signals of overpressure coming from the pressure control switch 60 and from the device sensing the endo-ocular pressure, actuates, if necessary, the electrovalves 51 and 61 (feed-back lines 72 and 73, respectively).

The operation of the infusion unit is thus based on the principle of feeding the infusion liquid from a containing tank under an adjustable and constant gas pressure, independently from the liquid head existing each time above the discharge outlet.

Clearly the plenum chamber 63 may each time be substituted for by a new tank, fulfilling the sterility requirements and fitted to the ducts 62 and 65 in a manner

**0121277**

known in the art.

It is lastly evident that the invention may be also applied outside of the ocular surgery, both in the surgical and in the therapeutical field, when the suction and/or the infusion in a controlled manner of biological liquids or of drugs must be carried out.

It is lastly to be meant the modifications and changes, both conceptually and structurally equivalent, are possible and foreseable without falling out of the scope of the invention.

CLAIMS                                    **0121277**

1. Automatic suction and pumping apparatus for using instruments, comprising a suction unit and an unit for infusion under pressure, characterized in that:

a)   said suction unit comprises a connection section to the outlet fitting of the using instrument adapted to establish a continuous flow connection with a duct of the using instrument, and a suction source in which a constant and precisely adjustable vacuum is generated;

b)   said unit for infusion under pressure comprises a feeding tank of the infusion liquid, feeding means for the feeding of a thrust gas under a desired and adjustable pressure to the said feeding tank, whereby said liquid is discharged from said tank at the pressure of said thrust gas independently from the hydraulic head of said liquid with respect to the discharge from said feeding tank, connection means between said discharge and an element of infusion under pressure connected to said using instrument, means for the detection of the environnment pressure at the discharge of said liquid from said infusion element and means for the control and adjustment of the pressure of said thrust gas fed to said feeding tank.

2. Apparatus according to claim 1, characterized in that said using instrument is a bistoury for ocular surgery, comprising an axial duct wherein an outwardly communicating suction opening is provided, said connection section being connected at one end with said axial duct and at the other end with said suction source.

3. Apparatus according to claim 2, characterized in that said connection section comprises a filter and a check valve.

4. Apparatus according to claim 3, characterized in that said connection section is of sterile and disposable type.

5. Apparatus according to claim 2, characterized in that said suction source consists of a hydraulic pump having the suction duct and the delivery duct connected as a closed circuit to a tank of liquid, a predetermined and adjustable neck being provided in said suction duct, said other end of said connection section being connected to the said suction duct.

6. Apparatus according to claim 5, characterized in that said hydraulic pump is actuated by an electrical motor, the speed of which is controlled and adjustable.

**0121277**

7. Apparatus according to claim 1, characterized in that said feeding means for the thrust gas comprise a line for the feeding of air or other gas under pressure, first pressure adjusting means, an electropneumatic device for the precision adjustment of said pressure and a duct for the feeding of said air or gas under pressure into a closed space in said tank above the infusion liquid, said tank having a liquid discharge connected with said infusion element.

8. Apparatus according to claim 7, characterized in that between said electropneumatic device and said feeding duct a pressure sensing switch and a quick discharge electrovalve are mounted.

9. Apparatus according to claim 7, characterized in that pressure sensing means are connected to said infusion element, said sensing means being in turn connected to a pressure transducer connected to a control and adjustment unit.

10. Apparatus according to claim 9, characterized in that said control and adjustment unit is connected to said pressure sensing switch.

11. Apparatus according to claim 10, characterized in that said control and adjustment unit comprises a comparator of the pressure as measured from said sensing means with a theoretical predetermined pressure value and electrical connection means for the control of said electropneumatic device, for the control of the air or gas feed under pressure and for the control of the quick discharge electrovalve.

0121277

**Fig.1**

**Fig.2**

Fig.3

Fig.4

## European Patent Office

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-3 812 855 (BANKO) <br><br> * Figures; column. 3, line 12 - column 5, line 8 * | 1-3,5, 7-11 | A 61 F 9/00 |
| Y | US-A-3 941 122 (JONES) <br><br> * Figures; claim 1; column 8, lines 59-65 * | 1-3,5, 7-11 | |
| A | US-A-4 108 182 (HARTMAN) <br> * Column 2, lines 51-53; figures * | 4 | |
| A | US-A-4 340 037 (LEWICKY) <br> * Column 12, lines 36-52; figures * | 6 | |
| A | US-A-4 184 510 (MURRY) | | **TECHNICAL FIELDS SEARCHED (Int. Cl. 3)** <br><br> A 61 F <br> A 61 M |
| A | US-A-4 117 843 (BANKO) | | |

The present search report has been drawn up for all claims

| Place of search | Date of completion of the search | Examiner |
|---|---|---|
| THE HAGUE | 05-07-1984 | STEENBAKKER J. |